# EUROPEAN PATENT SPECIFICATION

(11) **EP 0 559 725 B1**
(45) Date of publication and mention of the grant of the patent: **23.06.1999**
(21) Application number: 92900152.7
(22) Date of filing: 13.11.1991
(51) Int. Cl.: C12N 15/62, C07K 14/435, C12P 21/02, C12N 1/21, D01F 4/00, C08J 5/18, C09K 19/38, G02F 1/35

(54) **STRUCTURAL PROTEINS FROM ARTIFICIAL GENES**
STRUKTURPROTEINE DURCH KÜNSTLICHE GENE
PROTEINES STRUCTURELLES PRODUITES A PARTIR DE GENES ARTIFICIELS

(30) Priority: 28.11.1990 US 618504
(43) Date of publication of application: 15.09.1993
(60) Divisional application: 97202058.0
(73) Proprietor: E.I. DU PONT DE NEMOURS AND COMPANY, Wilmington Delaware 19898 (US)
(72) Inventor: HOESS, Ronald, Herman, Chadds Ford, PA 19317 (US); O'BRIEN, John, Philip, Oxford, PA 19363 (US); SALEMME, Francis, Raymond, Kennett Square, PA 19348 (US)
(74) Representative: Jones, Alan John
(86) International application number: US9108245
(87) International publication number: WO9209695

(56) References cited:
- WO-A-90/05177
- NUCLEIC ACIDS RESEARCH, Vol. 9, No. 5, 11 March 1981, Arlington, Virginia, US, pages 1229-1240, J. HERISSE et F. GALIBERT, "Nucleotide Sequence of the EcoRI E Fragment of Adenovirus 2 Genome".
- GENE, Vol. 81, 1989, Amsterdam, NL, pages 267-274, C. CHATELLARD et J. CHROBOCZEK, "Synthesis of Human Adenovirus Type 2 Fiber Protein in Escherichia Coli Cells".
- EMBO JOURNAL, vol. 2, no. 8; Green et al, pp. 1357-1365

## Description

### Field of the Invention

This invention relates to the production of structural proteins by cloning and expressing artificial genes in bacteria. The proteins produced by the instant invention are useful as spinnable fibers.

### Technical Review

The ability to clone specific DNA sequences in bacteria has opened the potential for the production of novel proteins by bacteria. The variety of proteins which can be produced is limited only by the imagination of the scientist and the as yet not understood limitations of the producing bacteria.

Several novel proteins have been made by cloning synthetic DNA in bacteria. These initial attempts have been to make proteins which mimic desired properties of proteins occurring in nature. This has been possible due to the widespread availability of sequence information of a number of proteins and genes.

WO 88/03533 discusses quite comprehensively the background of cloning synthetic genes and discloses the incorporation of repetitive oligomeric DNA sequences (gagags) into synthetic genes, the cloning of the genes into bacteria, and the production of several silk-like proteins, elastomeric-like proteins and mixtures of the two. This reference is hereby incorporated by reference.

European Patent Application 0 294 979 also discloses the cloning of an artificial gene comprising DNA sequence encoding repetitive GAGAGS units to produce silk-like structural proteins based on the protein fibroin of the silk fiber.

Doel et al., Nucleic Acids Research, 8(20): 4575-4592 (1980) disclose the cloning in *E*. *coli* and the subsequent expression of two synthetic dodecanucleotides with repeating DNA sequences encoding the dipeptide aspartyl-phenylalanine. There was an apparent size limit to the cloned DNA sequence of about 900 base pairs.

Gupta et al., Biotechnology September 1983: 602-609, disclose cloning a DNA sequence consisting of repeats of a dodecanucleotide. They were unsuccessful in getting a product when the insert exceeded 120 base pairs. They stated that either there was a selection against inserts larger than 120 base pairs in *E. coli,* or there is an excision event which deletes part of the cloned synthetic DNA.

Kempe et al., Gene 39: 239-245 (1985) disclose a vector system for obtaining high yields of multiple copies of synthetic genes. The authors utilize 5 copies of a synthetic SP gene fused to the lac Z gene. Each copy encodes the production of Arg-Pro-Lys-Pro-Gln-Gln-Phe-Phe-Gly-Leu-Met-NH₂.

Goldberg et al., Gene, 80: 305-314 (1989) disclose the cloning in *E*. *coli* and subsequent expression therein of a synthetic gene for the collagen analog.poly(Gly-Pro-Pro). The authors point out that the size of their largest DNA insert was about 450 base pairs. The authors further point out that large segments of highly-repeated DNA may be unstable in *E*. *coli* even in the presence of a *rec A* mutation. They also found that 80% of the collagen-like peptide at 41°C was degraded in about 40 minutes.

Chatellard et al., Gene, 81: 267-274 (1989) disclose the cloning and expression in *E. coli* of the gene encoding the trimeric fiber protein of human adenovirus type 2. They used a gene expression system based on bacteriophage T7 RNA polymerase. While fiber protein was produced to the extent of about 1% of total host-cell protein, there was a toxic effect of the fiber protein on the growth of the bacteria which was diminished by the addition of glucose to the medium and by maintaining the pH above 7. The product was insoluble in aqueous salt solutions and buffers in the absence of detergent or urea. The authors point out reasons to think the protein fiber product did not fold correctly.

The Adenovirus type 2 fiber gene has been sequenced by Herisse et al., Nucleic acids Research, 9: 1229-1240 & 4023-4042 (1981).

Green et al., The EMBO Journal 2(8): 1357-1365 (1983) by analyzing the sequence of the adenovirus type 2 fiber protein reported that a 15-residue segment repeats over more than approximately 300 residues. They indicate that each 15-residue segment contained two short β-strands and two β-bends.

McGrath et al., A CUMIRP Progress Report, July 15, 1989, Genetically Controlled Synthesis of Novel Polymeric Materials, prepare amino acid sequences through cloning and expression of genes.

Klein et al., Plasmid 3, 88-91 (1980) describe a rapid microscale technique for Isolation of Recombinant Plasmid DBA Suitable for Restriction Enzyme Analysis.

S. C. Stinson, C&EN, Biotechnoloay Providing Springboard to New Functional Materials, July 16, 1990, pp. 26-32, describes the use of repetitive amino acid sequences to make polymers useful for cell growth research and medical and dental needs.

Huang, Frank F., and Chokyun Rha, Protein Structures and Protein Fibers - A Review, Polymer Engineering and Science, February, 1974, Vol. 14, No. 2, reviews the biochemistry and stereochemistry of proteins related to fiber formation.

### SUMMARY OF THE INVENTION

This invention comprises a DNA sequence, and a protein encoded by said DNA sequence consisting of from 14 to 52 sequential multiples of

SGLDFDNNALRIKLG (SEQ ID NO:3).

The primary object of the invention is to produce polypeptides of defined sequence that spontaneously organize into fibrillar structures.

Another object of the invention is to produce synthetic genes and by genetic engineering means, use them to produce proteins which form cross-beta sheets.

A further object is to use said proteins as liquid crystals and to produce unique fibers.

### DETAILED DESCRIPTION OF THE INVENTION

Neither the DNA sequences nor the proteins of this invention occur in nature. The terms "polypeptide" and "protein" are used interchangeably herein. The proteins are the products encoded by a synthetic oligonucleotide construct which comprises 1) a leader sequence, 2) from 14 to 75 repeats of a synthetic oligonucleotide, and 3) a terminator sequence, said construct being operably linked to a promoter and expressed in bacteria. As discussed above, the primary object of the invention is to produce polypeptides of a defined sequence that spontaneously organize into fibrillar structures. It is believed that the polypeptide chains, as synthesized, first exist transiently as flexible polymers, but as a consequence of specific features of its amino acid sequence, each polymer spontaneously folds into a regular, chain-folded cross-beta sheet, stabilized by hydrogen bonds formed between the amide groups of the polypeptide backbone. The intermediate sheets then associate into aggregates composed of multiple, aligned copies of the cross-beta sheets, forming stiff microfibrils. When solutions of these stiff molecules are concentrated, they form lyotropic liquid crystalline phases (a well known property of such structures which demonstrate anisotropic character). Because lyotropic liquid crystals contain relatively large aligned aggregates of the microfibrils, such solutions can in turn be spun to produce fibers characterized by a high degree of molecular orientation. Such materials find utility as fibers or other materials in applications that benefit from incorporating a high degree of molecular organization to impart useful physical properties (e.g., non-linear optical properties). The polypeptides encoded by the synthetic sequences of this invention form such structures. Potential applications of these proteins include their use as stiff, high-modulus fiber reinforcements for biocompatible medical implants, and, because they are non-centrosymmetric materials as components for optical, electrical, or magnetic signal transfer and processing devices.

An effective method of carrying out the present invention is described below. The amino acid sequence of the desired synthetic protein is first determined. An oligonucleotide encoding those amino acids is then synthesized. Methods to synthesize oligonucleotides are well known to an ordinary person skilled in the art. Since the DNA code for amino acids is degenerate, the codons selected to make up the oligonucleotide are those which appear in highly-expressed proteins of *E. coli.* Also, the oligonucleotides are designed such that when they are hybridized to a complementary oligonucleotide, the ends of the resulting double-stranded molecule will be single-stranded and nonpalindromic. An oligonucleotide of the desired sequence and its complement are then synthesized, purified and hybridized using conventional methods. Said oligonucleotides are then phosphorylated at their 5' termini by means of an appropriate reaction using polynucleotide kinase (Maniatis et al., Molecular Cloning, A Laboratory Manual, Cold Spring Harbor Laboratory, Box 100, Cold Spring Harbor, NY). The resulting phosphorylated, hybridized DNA oligonucleotides are then incubated with T4 ligase and thereby linked together to form concatemers of varying lengths. Because the ends of the oligonucleotides are non-palindromic, the oligonucleotides are joined in a head to tail fashion with respect to one another. The resulting concatemers of varying lengths can be cloned directly, or they can first be separated into concatemers of different lengths prior to being cloned.

The purified concatemeric oligonucleotides are then ligated into a suitable expression vector containing a gene which codes for resistance to a selective substance such as ampicillin, such as pRHF-1 and pRHF-4. The vector comprises (1) a strong conditional promoter upstream of the synthetic gene to direct high levels of expression under controlled conditions, such as the bacteriophage lambda P_{L} promoter or the bacteriophage T7 gene 10 promoter, (2) a sequence downstream of the promoter that would contain the signals necessary for the translation start of the synthetic gene, (3) a unique restriction site downstream of the translational start that would allow the insertion of the concatemeric oligonucleotide in the correct orientation and proper reading frame such that translation will result in protein with the desired amino acid sequence.

Following ligation of the oligonucleotide into the appropriate vector, the resulting construct is transformed into a suitable CaCl₂-treated bacterial host strain (Maniatis et al., Molecular Cloning, A Laboratory Manual, Cold Spring Harbor Laboratory, Box 100, Cold Spring Harbor, NY). If expression is not desired from the T7 promoter, the host may be any *E. coli* strain that is defective for the *recA* gene *(recA*^{*-*}*).* The *recA*^{*-*} defect inactivates the host's homologous recombination system and thus insures against loss of the tandem repeated copies of the oligonucleotide by recombination. *E. coli* strains such as DH5Δlac and JM109 would be suitable. For conditional expression from the T7 promoter the vector must be transformed into the *E. coli* strain BL21 or derivatives thereof which contain a chromosomal copy of the T7 RNA polymerase gene under the control of the *lac* promoter (Studier et al., J. Mol. Biol. 189: 113-130 (1986)). For vectors in which the bacteriophage lambda PL promoter is used, the vector must always be transformed into a bacterial strain which carries the lambda cI857 repressor gene. An example of such a strain would be OR1265. The promoter is activated by raising the temperature of the cells to 42°C which inactivates the repressor.

Following their transformation, the bacteria are plated on medium containing 150 µg/ml ampicillin. Since all of the above mentioned vectors contain the gene that expresses β-lactamase, only those cells receiving the plasmid will be ampicillin resistant and grow in the presence of ampicillin. To enrich for vectors that have the oligonucleotide inserted, the vectors are treated prior to ligation with calf intestinal phosphatase (Maniatis et al., supra). Treatment with alkaline phosphatase removes the PO₄ from the 5' termini of the vector and thus eliminates reclosure of the vector without an oligonucleotide insert since 5' termini without PO₄ is not a substrate for T4 DNA ligase.

To find out which transformants have an oligonucleotide insert, a number of different transformed colonies are grown in Luria Broth (LB) containing 100 µg/ml ampicillin until the cultures reach the stationary phase of growth. The plasmid DNA from each culture is then extracted from the harvested cells by the procedure of Holmes et al., Anal. Biochem. 114: 193 (1981). Plasmid DNA is separated according to size by electrophoresis on 1% agarose gels (Maniatis, supra). The larger plasmids, (i.e., those plasmids with large inserts) are selected. The size of the DNA insert in each plasmid is estimated by cleaving the DNA with a restriction enzyme (according to suppliers instructions) that cleave near the boundaries of the insert but not within the insert. This DNA is then electrophoresed on a polyacrylamide gel (i.e., 5%) with appropriate molecular weight standards so that the size of the insert can be determined. Those plasmids with larger insert sizes (calculated to have about 14 to 26 repeating units of the original oligonucleotide) are selected.

Plasmids that are identified as having larger inserts are then transformed into CaCl₂ treated *E. coli* strain BL21 or any *E. coli* strain that contains the T7 RNA polymerase gene. Transformants are selected by plating the cells on LB agar plates containing 150 µg/ml of ampicillin. Single colonies are selected and grown in LB broth containing 100 µg/ml ampicillin until the culture reaches an optical density (OD₆₀₀) of 0.5. Isopropylthio-β-galactoside (IPTG) is added to a final concentration of 0.5 mM to induce the T7 RNA polymerase gene. This in turn leads to the expression of the synthetic gene since its promoter is only recognized by the T7 RNA polymerase. Conditional expression of the synthetic genes is necessary since the gene products appear toxic to the *E. coli* producing them. Cells are grown at 37°C for from 2-4 h in the presence of IPTG. Following this induction period the cells are collected by centrifugation. Production of the synthetic gene product is monitored by analyzing the proteins from samples of the induced bacteria on 12.5% SDS-polyacrylamide gels (Laemmli, Anal. Biochem. 114: 193 (1981)) and staining them with Coomassie Blue dye. The synthetic proteins migrate with the size predicted from calculating the number of oligonucleotide inserts that comprise the gene. The resulting proteins encoded by the cloned synthetic genes of this invention can be recovered and purified by conventional means preferably by means of centrifugation and chromatography on sepharose gels. The resulting proteins can be formed into fibers by conventional spinning methods known to those skilled in the art. In general, polypeptides are soluble in a limited number of solvents and are typically spun from acid solutions. Heating may be required to dissolve the polypeptide in certain solvents. However, polypeptides having long side chains are frequently spun from organic solutions. While the use of hexafluoroisopropanol (HFIP) as a solvent for purification of synthetic polyamides and polypeptides, has been disclosed in Teti, U.S. Patent No. 3.696,058 and Hayashi, U.S. Patent No. 4,594,409, it has now been found that HFIP is useful as a spinning solvent for polypeptides. As a solvent to spin from, HFIP has the advantage that stable solutions of polypeptides, wherein the polypeptides undergo no measurable degradation, are formed.

Solutions of fiber-forming polypeptides in the solvent hexafluoroisopropanol may be spun into fibers in wet spinning, air-gap spinning, and dry spinning processes. Polypeptides having molecular weights greater than 20,000 are useful in this invention.

Wet spinning, air-gap spinning, and dry spinning processes are well known in the art. Descriptions of wet and dry spinning processes are given in Billmeyer, Textbook of Polymer Chemistry, 1957, pp. 388-395. An example of an air-gap spinning process is disclosed in De Lucca, et. al., U.S. Patent No. 4,857,403. Air-gap spinning typically is used instead of wet spinning when an improvement in physical properties can be achieved. This is typically the case when the spinning solutions comprise liquid crystalline solutions, where alignment of the crystalline domains occurs during attenuation of the solution in the air gap.

According to the current invention, spinning solutions are prepared by dissolving approximately 5-30 wt % of the polypeptide in the solvent hexafluoroisopropanol (HFIP). Urea (0.5-25%) may be added to enhance spinning properties of the polypeptide in HFIP. The spinning solutions are typically prepared at room temperature. However, dissolution of polypeptides in HFIP is more rapid if the solutions are heated slightly.

In a wet spinning process, the spinning solution is extruded directly into a coagulating bath. The coagulant used may be any solvent in which the HFIP solvent is soluble, but in which the polypeptide is insoluble. Examples of useful coagulants include water, methanol, ethanol, isopropyl alcohol, and acetone. Generally, methanol has been found to be the preferred coagulant for certain polypeptide spinning solutions. However, acetone was found to be the preferred coagulant for some of the polypeptides of this invention. In certain instances, the resultant fibers may be dried and subsequently hot drawn to obtain an improvement in physical properties. When the polypeptide fibers are not amenable to hot drawing, it has been found that it is preferable to cold draw the fibers while still wet with the coagulant. When the fibers are allowed to dry prior to cold drawing, limited drawing occurs and the fibers have a tendency to break.

In an air-gap spinning process, the spinning solution is attenuated in an air gap before entering the coagulant bath. For lyotropic spinning solutions, the stretching that occurs in the air gap can result in alignment of crystalline domains which are frozen in an oriented arrangement in the coagulation bath, which often results in improved tensile properties over wet-spun fibers. Coagulants useful in air-gap spinning processes are the same as those that are useful for wet spinning.

In a dry spinning process, instead of spinning into a coagulating bath, the solvent is evaporated, for example by spinning into hot air.

### EXAMPLE 1

### CREATION OF A VECTOR PRODUCING A PROTEIN COMPRISING MULTIMERS OF SGLDFDNNALRIKLG

To produce a protein comprising multimers of SGLDFDNNALRIKLG, two oligonucleotides were synthesized, and purified. They were:

(1) 5'CTGGTCTGGACTTTGACAACAACGCTCTGCGTATCAAGCTGGGCT3'

(2) 5'CAGCTTGATACGCAGAGCGGTGTTGTCAAAGTCCAGACCAGAGCC3'

Each oligonucleotide (2 µg) was incubated with 10 units of polynucleotide kinase (Boehringer Mannheim, P.O. Box 50414, Indianapolis, IN 46250) in a 10 µL reaction volume containing 50 mM Tris-HCl (pH 7.5), 10 mM MgC12, 5 mM β-mercaptoethanol, 0.1 mM spermidine, 0.1 mM Na₂EDTA and 1.0 mM ATP. The polynucleotide kinase transfers PO₄ to the 5' termini of the oligonucleotides. The oligonucleotides must have a 5' PO₄ to serve as a substrate for DNA ligase in subsequent steps. Following 1 h at 37°C the two reactions were combined and further enzymatic activity terminated by heating the resulting mixture for 10 min at 70°C. The mixture was then allowed to slowly cool to room temperature wherein the oligonucleotides hybridized with each other.

Vector pRHF-4 was digested in 10 mM Tris-HCl (pH 7.5), 10 mM MgCl₂, 50 mM NaCl with the restriction enzyme *Ban*II which cuts at a unique site in the vector thereby linearizing the vector. Following this digestion, the reaction was phenol-extracted to separate plasmid DNA from the restriction enzyme and other proteins, and the DNA was then collected by precipitation with ethanol. Hybridized phosphorylated oligonucleotide (0.2 µg) and BanII linearized vector (∼20 ng) were incubated together in a 20 µl reaction volume containing 50 mM Tris-HCl (pH 7.5), 10 mM MgCl₂, 5 mM spermidine, 0.1 mM Na2EDTA, 1 mM ATP overnight at 16°C. The ligation reaction was terminated by heating the reaction mixture for 10 min at 70°C. The mixture was then used to transform 100 µl of *E. coli* (strain DH5Δ lac) made competent by the CaCl₂ treatment as known by those skilled in the art. The transformed bacteria were then plated on Luria Broth (LB) agar containing 150 mg/ml of ampicillin. The bacteria containing agar plates were incubated overnight at 37°C. Since pRHF-4 carries the gene for resistance to ampicillin, only those bacteria transformed with the plasmid will grow in the presence of ampicillin. Eighteen transformed colonies were selected and individually grown in 10 ml of LB broth containing 100 µg/ml ampicillin. When the cultures reached an absorbance (at OD₆₀₀) of about 0.8, chloramphenicol was added to a final concentration of 100 µg/ml and the culture was further incubated at 37°C overnight. Plasmid DNA was extracted from the bacteria of each culture (Klein et al. Plasmid 3: 88-91 (1980)) and digested with the restriction enzyme *Hae*III for 1 h at 37°C in 10 mM Tris-HCl (pH 7.5), 10 mM MgCl₂, and 50 mM NaCl. The digested DNA was electrophoresed on a 5% polyacrylamide gel. We determined the size of the restriction fragments and calculated the size of the inserted oligonucleotide. Clones 5 and 9 contained 2 and 5 concatemeric multiples of the monomeric oligonucleotide, respectively. These plasmids are pRHF-5 and pRHF-6, respectively.

Plasmid pRHF-5 (15 µg) was digested with the restriction enzyme *Ban*II for 2 h at 37°C in a 200 µl reaction volume. The digested DNA was separated from other proteins by phenol extraction and collected by precipitating it with ethanol. The resulting DNA was electrophoresed on an 8% polyacrylamide gel and the DNA visualized after staining with ethidium bromide. As a result of the BanII digestion, the cloned oligonucleotide was cleaved to a monomer. This band of DNA was collected from the gel by electroelution and concentrated by ethanol precipitation. The isolated DNA was subjected to a ligation reaction as described above. When the incubation of the ligation mixture had proceeded for 30 min, DNA from pRHF-4, linearized with BanII, was added to the mixture and the incubation was continued overnight. As before, the ligation reaction was terminated by heating the mixture for 10 min at 70°C. The resulting DNA mixture was used to transform competent *E. coli* (DH5 *lac*) as before. As before, the resulting bacteria were plated on LB agar plates containing 150 µg of ampicillin. Thirty transformants were each inoculated into 1 ml of LB containing 150 µg of ampicillin. The plasmid DNA of each culture was obtained, electrophoresed on a 1% agarose gel and stained with ethidium bromide. Clones 3, 8, 9, and 23 appeared to have greater multiples of the monomeric insert. Larger cultures of clones 3, 8, 9, and 23 were prepared and the plasmid DNA from each was obtained. Each plasmid DNA was digested with the restriction enzyme *Hae*III and the digested DNA was electrophoresed on a 3.5% polyacrylamide gel. Clones 3 and 9 appeared to have the largest inserted DNA fragment. The number of concatemeric multiples of the monomer oligonucleotide in clones 3 (pRHF-7) and 9 (pRHF-8) were 18 and 23, respectively.

Plasmid DNAs pRHF-7 and pRHF-8 were transformed into competent *E. coli* bacteria strains BL21/LysE *recA* and BL21/lys S *recA*. A single colony of each of the transformed bacterial strains was grown in LB containing 150 µg/ml of ampicillin until the culture reached on OD600 of about 0.3. A sample of bacterial cells (uninduced) was removed at this time, pelleted by centrifugation, and boiled for 5 min in sodium dodecylsulfate (SDS) loading buffer. To the remainder of each culture, IPTG (isopropylthio-β-galactoside) was added to a final concentration of 0.5 mM followed by 3 h of further incubation. A sample of bacterial cells (induced) was then removed, pelleted by centrifugation, and boiled for 5 min in SDS loading buffer. The proteins obtained from both the uninduced and the induced bacterial cultures were electrophoresed on a 12.5% SDS polyacrylamide gel. Proteins were visualized on the gel by staining them with Coomassie blue. The apparent molecular weight of the protein from the induced bacteria transformed with pRHF-7 was about 30 kilodaltons, while that of the DNA from the induced bacteria transformed with pRHF-8 was about 42 kilodaltons. Hereafter, this protein will be referred to as the 40,000 dalton protein. A more accurate size determination of the DNA insert in pRHF-8 was done by digesting the DNA from that plasmid with the restriction enzyme *DdeI* and electrophoresing the DNA on a 3.5% polyacrylamide gel. The inserted fragment of DNA was found to consist of 26 concatemeric multiples of the monomeric oligonucleotide which agrees with the molecular weight of the induced protein.

### LARGE SCALE PRODUCTION OF THE 40.000 DALTON PROTEIN

Colonies picked from an agar plate containing transformed *E. coli* strain BL2l/pRHF8, pLysS were grown as separate cultures for 6 h in 5 ml of the culture Medium A, whose composition is shown below. Each resulting culture was diluted 1:1 in medium B, whose composition also is shown below, and then frozen and stored at -70°C. Prior to freezing, an aliquot from each culture was placed in 40 ml of medium A, grown at 36°C to a turbidity of 1.0 (at OD₆₀₀) , then induced by adding 40 mg of (IPTG, Boehringer Mannheim,
Indianapolis, IN 46250). The induced culture was incubated an additional 2.5 h, then 1 ml aliquots were centrifuged. Pellets were assayed for the quantity of protein product with a molecular weight of 40,000 daltons by using laser scanning densitometry on SDS-PAGE gels. SDS-PAGE means polyacrylamide gel electrophoresis wherein the gel contains sodium dodecylsulfate. The culture which produced the most protein was selected.

An inoculum (about 50 µl) from its corresponding frozen culture was used to initiate a new culture by adding it to 5 ml of Medium A in a 15 ml tube and incubating the resulting culture for six hours. An aliquot (0.5 ml) of this culture was added to 500 ml of Medium A in a 2-liter flask and the resulting culture was incubated about 14 hours at 36°C, being continuously swirled at 150 rpm, until the culture reached a turbidity (OD₆₀₀) of 2.7. At this turbidity, the concentration of cell protein was 0.9 g/l. The 500 ml culture served as an inoculum and was added aseptically to a 16 liter fermenter containing 9.5 liters of Medium A. The 10 liter culture was incubated for 5 h at 36°C, (pH 7.1, dissolved oxygen 60% atm), to a turbidity (OD₆₀₀) of about 15 (cell protein 3.5 g/l). The culture was induced by adding 12 g of IPTG, incubated an additional 3.5 hours, then chilled to 9°C. The culture was centrifuged at 4°C, and the resulting paste of bacteria was recovered and stored at -70°C. Prior to the centrifugation, the concentration of cell protein in the culture was 4.1 g/l; and the turbidity (OD₆₀₀) was 29. By densitometrically scanning a SDS-PAGE Coomassiestained gel, it was determined that the engineered 40,000 dalton protein represented 9.7% of the total cell protein. Further calculation indicated that the resulting paste of bacteria contained about 3.9 g of the 40,000 dalton protein. Estimates of 40,000 dalton protein are conservative, since the protein does not stain well with Coomassie.

### PURIFICATION OF THE 40.000 DALTON PROTEIN

Cell paste (300 g) was suspended in 3 liters of Tris (hydroxymethyl) aminomethane buffer at pH 8.0 which contained ethylenediaminetetraacetate (EDTA) at a concentration of 10 mM, phenylmethylsulfonyl fluoride (PMSF) at a concentration of 1 mM and 100 mM NaCl and stirred at room temperature until a uniform suspension was obtained. The bacterial cells were then lysed by adding 1.5 gram of lysozyme to the suspension and stirring it for an additional 30 min.

When the cells were lysed, the suspension became viscous because of the released DNA. Therefore, the suspension was stirred for 30 min with deoxyribonuclease (15 mg) in the presence of 50 mM Mg(Cl)₂ whereupon the viscosity of the suspension decreased. The suspension was clarified by centrifugation at 25,000 x g for 2 h. Inclusion bodies were prepared by first washing the resulting pellet twice with a detergent solution (50 mM Tris (hydroxymethyl) aminomethane, 5 mM ethylenediaminetetraacetate (EDTA), 1 mM phenylmethylsulfonyl fluoride (PMSF), 2% Triton X-100, pH 8.0) and then with a solution of 4 M urea. PMSF was added each time just prior to use. The 4 M urea solution was made from a freshly prepared 9.6 M stock solution buffered with 50 mM sodium Phosphate at pH 8.0

The pellet was then suspended in 3 liters of detergent solution and stirred for 2 h at room temperature, then the suspension was centrifuged at 25,000 x g for 2 h. The detergent wash as above was repeated on the resulting pellet. The pellet weighed 132 g.

The 132 g pellet was suspended in 1.32 liters of 4 M urea buffered with 50 mM sodium phosphate, pH 8.0, and stirred for 2 h at room temperature. The suspension was clarified by centrifugation as described above and the resulting pellet recovered. The pellet was resuspended and recentrifuged as just described. After this second wash, the pellet weighed 90 g.

A major portion of the pellet (80 g) was suspended in 800 ml of 6 M guanidine hydrochloride buffered with 50 mM Tris at pH 8.0 and stirred overnight at room temperature. The suspension was then centrifuged at 25,000 x g for 2 h at 10°C. The supernatant fluid was exchanged into 6 M urea buffered with 20 mM 2-(N-Morpholino)ethanesulfonic acid (MES) at pH 6.0 using a 6-liter Bio-Rad P-6DG gel desalting column (9 x 94.3 cm). The 800 ml sample was pumped through the desalting column at a flow rate of 1 l/h. The first two liters were discarded. The next liter which contained the void volume was collected and again clarified by centrifuging at 25,000 x g for 2 h at 10°C. The supernatant fluid was passed through a two liter QFF sepharose column (9 x 31.4 cm) at a flow rate of 2 l/h. A volume (1.8 liters) of the effluent was collected and dialyzed exhaustively against distilled water at 4°C. The dialyzed sample was shell frozen and lyophilized. The weight of the lyophilized sample was 5.5 g.

SDS-PAGE electrophoresis of an aliquots of the sample showed one predominant band with a molecular weight of about 40,000 daltons. The protein was judged to better than 95% pure with respect to contaminating proteins by this criterion. It was noted that significant amounts of urea were present in the lyophilized sample of the 40,000 dalton protein. The amino acid composition agreed, within experimental error, with that determined from the DNA sequence of the cloned DNA. The N-terminal sequence is in perfect agreement with the predicted sequence except for the missing N-terminal methionine.

| MEDIA: | |
|---|---|
| Medium A (grams of chemical) | |
| Solution A | |
| K₂HPO₄ | 19.5 |
| NaH₂PO₄ | 10.3 |
| autoclave in 0.5 liter of deionized water | |

| Solution B | |
|---|---|
| CaCl₂ | 0.25 |
| glucose 200 (flask & tube) or 300 (fermenter) autoclave in 1 liter of deionized water. | |

| Solution C | |
|---|---|
| (NH ₄)₂SO₄ | 3.0 |
| MgSO₄ | 4.5 |
| proline | 3.7 |
| Na citrate.H₂O | 0.43 |
| FeSO₄.H₂O | 0.25 |
| thiamine | 0.30 |
| Trace elements 10 ml PPG2000 5 ml | |
| Casamino Acids 100 g (tube & flask) or 200 g | |
| (fermenter) all in 8 liters of deionized water autoclave in carboy or in fermenter | |

| Trace Elements Solution (g/l) | |
|---|---|
| ZnSO₄ | 4.5 |
| CuSO₄ | 2.25 |
| MnSO₄ | 2.25 |
| CoCl₂ | 0.0307 |
| NH₄MoO₄ | 0.090 |
| H₃BO₄ autoclave | 0.15 |

When cool, combine solutions A,B, C and trace elements solution, and add filter-sterilized ampicillin solution to a concentration of 50 mg/l of ampicillin.

| | |
|---|---|
| Medium B (g/l for 2X concentration) | |
| K₂HPO₄ | 13.6 |
| KH₂PO₄ | 3.6 |
| Na₃citrate.H₂O | 0.9 |
| MgSO₄.7H₂O | 0.18 |
| (NH₄)₂SO₄ | 1.8 |
| glycerol | 88.0 |
| Autoclave, then store at 4°C. | |

### DEMONSTRATION OF ANISOTROPY OF THE 40.000 DALTON PROTEIN

A solution containing 19.1% of the lyophilized preparation of the 40,000 dalton r-protein and 5.5% urea was prepared by combining 0.0760 g total solids and 0.2330 g HFIP in a heat-sealed polyethylene container. Intermittent vigorous mixing followed by standing overnight at room temperature produced a stiff, pasty, non-flowing but deformable (yield stress or Bingham plastic behavior), opaque, translucent solution. Appearance and consistency were comparable to that of the stopcock grease or high vacuum grease used in assembling laboratory glassware. A sample of the solution was placed between crossed polarizing filters (set 90 degrees apart) in the optical train of a light microscope and was examined in the resulting dark field. The sample was strongly birefringent, showing zones which brightened and darkened as the sample was rotated in the plane of the microscope stage, with a grainy, sandpaper-like texture. The solution was characterized as anisotropic (liquid crystalline) on the basis of its rheology, overall appearance, and optical properties.

A solution containing 9.9% of the lyophilized preparation of the 40,000 dalton r-protein and 2.9% urea was prepared by combining 0.0780 g total solids and 0.5315 g hexafluoroisopropanol (HFIP) in a heat-sealed polyethylene container. Intermittent vigorous mixing following by standing overnight at room temperature produced a pasty, opaque, translucent solution. As prepared, the solution was smooth and easy to mix, but it showed a definite yield stress (Bingham plastic) rheology (no flow until a certain minimum force was applied, smooth flow thereafter; minimum force for flow greater than the solution's own weight). A sample of the solution was placed between crossed polarizing filters (set 90 degrees apart) in the optical train of a light microscope and was examined in the resulting dark field. The sample was strongly birefringent, showing zones which brightened and darkened as the sample was rotated in the plane of the microscope stage, with the characteristic texture of an anisotropic (liquid crystal) solution of stiff, rod-like molecules.

A solution containing 3.7% of the lyophilized preparation of the 40,000 dalton r-protein and 1.0% urea in HFIP was prepared by combining 0.0524 g total solids and 1.0677 g HFIP in a heat-sealed polyethylene container. Intermittent vigorous mixing following by standing overnight at room temperature produced a thin, readily pourable, clear solution. A sample of the solution was placed between crossed polarizing filters (set 90 degrees apart) in the optical train of a light microscope and was examined in the resulting dark field. The sample was optically inactive and could not be seen in the dark field. The solution was characterized as isotropic on the basis of its rheology, overall appearance, and optical properties.

On again standing overnight at room temperature, the clear 3.7% solution of the 40,000 dalton r-protein in HFIP became turbid and opaque. However, there was no change in the appearance of the solution in the dark field microscope and no significant change in solution rheology. The solution remained isotropic. The development of turbidity was attributed to the aggregation (self-assembly) of the protein polymer in solution.

### FIBER FORMATION WITH THE 40,000 DALTON PROTEIN

(A) A solution of 7.5% (by weight) of the lyophilized preparation of the 40,000 dalton polypeptide plus 2.2% (by weight) of urea in HFIP was prepared by adding the solvent to the dry ingredients in a heat-sealed polyethylene packet, mixing thoroughly by kneading the packet by hand, and allowing the mixture to stand overnight at room temperature. The solution showed the translucent, opalescent appearance and yield-stress rheology which is characteristic of liquid crystalline solutions. A sample of the solution was placed between crossed polarizing filters which were set apart at 90 degrees in the optical train of a light microscope and the sample was examined in the resulting dark field. The sample was found to be strongly birefringent, showing zones which brightened and darkened as the sample was rotated in the plane of the microscope stage.

The solution was filtered through a stainless steel screen pack consisting of 50, 325, and 50 mesh (mesh is according to ASTM Standard E-11-61. see Lange's Handbook of Chemistry, 13th edition, pages 11 & 12) screens prior to loading into a syringe for wet spinning. The syringe was capped and centrifuged to disengage any air bubbles trapped in the solution. A syringe pump was then used to force the solution out of the syringe through a 0.005" diameter x 0.010" length orifice in a stainless steel fitting directly into a container of acetone at room temperature. Syringe pump speed was set to deliver 0.0034 ml/min of solution. The filament which formed as the solution was extruded into the acetone was allowed to fall freely and to coil on itself at the bottom of the acetone filled container.

After at least 10 minutes of coagulation in the acetone, the filament was removed and allowed to dry in air at room temperature to produce an 18 denier fiber with a tenacity of 0.4 gpd, an elongation of 34 %, and an initial modulus of 16 gpd.

In a separate experiment, alternatively, the wet filament was drawn up to 2 times its original length as it was removed from the acetone. Wet drawing to 1.5 times original length followed by air drying produced a 5 denier fiber with a tenacity of 1.5 gpd, an elongation of 16%, and an initial modulus of 45 gpd.

Dried fibers prepared according to the process described above were drawn to 2 - 3 times their original length while passing over a 200°C hot pin to produce a 5 denier fiber with a tenacity of 2.6 gpd, an elongation of 15%, and an initial modulus of 44 gpd.

(B) A solution containing 11.9 wt% of the about 40,000 dalton polypeptide and 4.0% urea was prepared in HFIP by adding the solvent to the dry ingredients in a heat-sealed polyethylene packet. Intermittent, vigorous mixing (kneading by hand) during five days at room temperature produced a thick, translucent solution having a yield-stress rheology characteristic of liquid crystal solutions.

The solution was transferred into a syringe fitted with 50, 325, 325, and 50 mesh screens. The syringe was capped and centrifuged to disengage air bubbles trapped in the solution. A syringe pump was then used to force the solution through the screen pack and out of the syringe through a 0.005 inch diameter x 0.010 inch length orifice in a stainless steel fitting. The syringe pump speed was set to deliver 0.068 ml/min of solution. The syringe, syringe pump, and steel fitting were arranged so that the jet of solution emerging from the orifice travelled down through a 0.5 inch air gap into a pan of acetone which was at room temperature. The filament produced by the coagulation of the jet in the acetone was collected at a rate of 12 ft/min by winding onto bobbins on a motor-driven windup and allowed to dry in the air at room temperature. After drying in air at room temperature, the fiber was removed from the bobbins and found to be 15.6 denier with a tenacity of 1.2 gpd, elongation of 9%, and an initial modulus of 58 gpd.

(C) The polymer solution described in (B) was spun in a dry-spinning process with the syringe, syringe pump, and steel fitting arranged so that the jet of solution emerging from the orifice fell through an air gap approximately 18 inches long across which air at room temperature was flowing to evaporate the HFIP. The syringe pump speed was set to deliver 0.0068 ml/min of solution. The partially dried filament at the bottom of the gap was wound onto metal mesh bobbins and allowed to dry completely in air at room temperature. The dry fiber was 24 denier, with a tenacity of 0.1 gpd, elongation of 37%, and an initial modulus of 1.5 gpd.

### PRESENCE OF CROSS-BETA SHEET MICROFIBRILS IN THE FIBERS MADE WITH THE 40,000 DALTON PROTETN

A spun and post drawn fiber was subjected to X-ray diffraction. The diffraction pattern was consistent with the presence of oriented cross-beta sheet fibrils in that the observed diffraction pattern of the oriented fiber corresponded to that of a model.

### EXAMPLE 2

### CREATION OF A VECTOR PRODUCING A PROTEIN COMPRISING MULTIMERS OF LSVOTSATLTVSDGK

To produce a protein comprising multimers of LSVQTSAPLTVSDGK, the following oligonucleotides were svnthesized: 
5' CTGTCTGTTCAGACCAGCGCGCCGCTGACCGTTTCCGACGGTAAG 3'
5' AGCTTACCGTCGGAAACGGTCAGCGGCGCGCTGGTCTGAACAGAC 3'
 Oligonucleotides (2 µg) of each strand were incubated with 10 units of polynucleotide kinase (Boehringer Mannheim, P.O. Box 50414, Indianapolis, IN 46250) in a 20 µl reaction volume containing 1.0 mM ATP, 50 mM Tris-HCl (pH 7.5), 10 mM MgCl₂, 5 mM β-mercaptoethanol, 0.1 mM spermidine and 0.1 mM Na₂EDTA. The reaction was incubated for 3 h at 37°C and then terminated by the addition of 20 µl of 0.01 M Tris-HCl (pH 7.5) and 40 µl of phenol. The aqueous phase following phenol extraction was then loaded onto a 1.0 ml Sephadex™ G-25 column equilibrated with 0.01 M Tris-HCl (pH 7.5). The oligonucleotides eluted in a volume of 49 µl. From this 8 µl was removed and used for DNA ligation. The ligation reaction consisted of oligonucleotides, 1.0 mM ATP, 50 mM Tris-HCl (pH 7.5), 10 mM MgCl₂, 5 mM β-mercaptoethanol, 0.1 mM spermidine, 0.1 mM Na₂EDTA, 40% polyethylene glycol (MW 8,000) and 400,000 units of T4 DNA ligase (New England Biolabs, 32 Tozer Rd, Beverly, MA 01915). The reaction was incubated for 30 min at room temperature and then SDS gel stop solution was added prior to loading the sample on a 0.8% agarose gel. Following electrophoresis, the gel was stained with ethidium bromide and oligonucleotide multimers in the 1-2 kb size range were excised from the gel. The gel slices containing the DNA were crushed in phenol and the extracted DNA was concentrated by ethanol precipitation. The DNA was then resuspended in 20 µl of 0.01 M Tris-HCl.

Vector pRHF-1 (2.4 µg) was digested with the restriction enzyme AccI in a reaction volume of 20 µl containing 50 mM Tris-HCl (pH 7.5) and 10 mM MgCl₂. The reaction was incubated at 37°C for 2 h and then incubated for an additional hour with 2 µl of a 1:50 dilution of calf alkaline phosphatase (Boehringer Mannheim, P.O. Box 50414, Indianapolis, IN 46250) to remove 5'-PO₄ from the ends of the vector. The reaction was then terminated by the addition of an equal volume of phenol to remove the proteins, and the DNA was then concentrated by ethanol precipitation. The concentrated DNA was then resuspended in a volume of 50 µl of 0.01 M Tris-HCl (pH 7.5).

The oligonucleotide multimers and vector DNA were then ligated together in a 25 µl reaction mixture containing 15 µl oligonucleotide multimers, 2.5 µl pRHF-1 AccI linearized vector DNA, 1.0 mM ATP, 50 mM Tris-HCl (pH 7.5), 10 mM MgCl₂, 5 mM β-mercaptoethanol, 0.1 mM spermidine and 0.1 mM Na₂EDTA. The reaction was incubated overnight at 16°C. The ligation reaction was terminated by heating the reaction for 10 min at 70°C. The mixture was then used to transform 175 µl of *E. coli* (strain DH5 *lac)* made competent by the CaC12 treatment as known by those skilled in the art. The transformed bacteria were then plated on LB agar containing 150 µg/ml of ampicillin. These plates were then incubated overnight at 37°C. Twenty-four transformed colonies were selected and individually grown in 10 ml of LB broth containing 100 µg/ml ampicillin. When the cultures reached an absorbance (OD₆₀₀) of -0.8, chloramphenicol was added to each to a final concentration of 100 µg/ml and the cultures were incubated further at 37°C overnight. Plasmid DNA was extracted from the bacteria of each culture (Klein et al., Plasmid 3: 88-91 (1980)) and digested with the restriction enzyme *EcoRI* for 1 h at 37°C in 10 mM Tris-HCl (pH 7.5), 10 mM MgCl₂, and 100 mM NaCl. The restriction digests were then electrophoresed on a 1% agarose gel and the DNA examined by staining with ethidium bromide. Clone 11, designated pRHF-11, had an estimated insert size of about 710 bp.

pRHF-11 DNA was transformed into competent *E. coli* bacteria strains BL21/LysE recA and BL21/lysS *recA.* A single colony of each of the transformed bacterial strains was grown in LB containing 150 µg/ml of ampicillin until the culture reached an absorbance (OD₆₀₀) of about 0.3. A sample of bacterial cells (uninduced) was removed at this time, pelleted by centrifugation, and boiled for 5 min in sodium dodecylsulfate (SDS) loading buffer. To the remainder of each culture, IPTG (isopropylthio-β-galactoside) was added to a final concentration of 0.5 mM followed by 3 h of further incubation. A sample of bacterial cells (induced) was then removed, pelleted by centrifugation, and boiled for 5 min in SDS loading buffer. The proteins obtained from both the uninduced and the induced bacterial cultures were electrophoresed on a 12.5% SDS polyacrylamide gel. Proteins were visualized on the gel by staining them with Coomassie blue. An induced protein from the transformed bacteria with a molecular weight of 20,000 daltons was observed. Hereafter this protein will be referred to as the 20,000 dalton protein.

*E. coli* strain BS583 F⁺/pRHF-11 was infected with the M13 phage IR-1 to produce single-stranded plasmid DNA according to the method of Zagursky et al., Gene 27: 183-191 (1984)). The single stranded DNA was sequenced using an EcoRI primer (New England Biolabs, supra) using the chain termination method of Sanger et al., Proc. Natl. Acad. Sci. USA 74: 5463-5467 (1977). Sequencing indicates that pRHF-11 contains 14 sequential multiples of the original oligonucleotide.

### LARGE SCALE PRODUCTION OF THE 20.000 DALTON PROTEIN

An inoculum (about 50 ul) from a culture of the *E. coli* transformed with pRHF-11 was added to 5 ml of medium B and incubated for about 6 h at 36°C in a 15 ml test tube. At this time, 0.5 ml of this first culture was added to 500 ml of medium B in a 2-liter flask and incubated about 14 h at 36°C, with continuous rotation at 150 rpm to a turbidity (OD₆₀₀) of 2.5. The 500 ml culture was then added to the 16-liter fermenter which contained 9.5 liters of medium C and the resulting culture was incubated 5 h at 36°C, (pH 7.2, dissolved oxygen 60% atm.), to a turbidity (OD₆₀₀) of 1.1. At this turbidity, the concentration of cell protein was 1.8 g/l. IPTG (2.5 g) was added. After an additional hour of incubation, another 7.5 g of IPTG was added, followed by two additional hours of incubation. The culture was then chilled to 18°C by passage of chilled water, centrifuged at 4°C, and the resulting paste of bacterial cells was stored at -70°C. Just prior to centrifuging the culture, the concentration of bacterial protein was 2.5 g/l. The turbidity (OD₆₀₀) of the culture was 15.6. The 20,000 dalton protein represented 2.2% of the total cell protein as determined by SDS-PAGE, Coomassie staining and subsequent laser densitometry. By calculation there was 0.64 g of the 20,000 dalton protein in the total cell paste, but later determination of purified 20,000 dalton protein showed that it was stained with only about 25% efficiency by Coomassie; thus this method underestimated the real amount of protein present.

| Medium B | (g/liter): |
|---|---|
| tryptone | 10 |
| yeast extract | 5 |
| NaCl | 10 |
| glucose | 10 |
| (Autoclaved separately and added when cool) Filter sterilized ampicillin was added to 50 mg/l. | |
| | |

| Medium C (for the fermenter (g/10 liters)): | |
|---|---|
| Solution A | |
| Na₂HPO₄ | 70 |
| KH₂PO₄ | 30 |
| NaCl | 5 |
| NH₄Cl | 10 |
| autoclave in 500 ml of H₂O. | |
| | |

| Solution B | |
|---|---|
| glycerol | 200 |
| CaCl₂ | 10 ml of 1M |
| autoclave in 1 liter of H₂O. | |
| | |

| Solution C | |
|---|---|
| casamino acids | 40 |
| peptone | 30 |
| yeast extract | 20 |
| proline | 0.2 |
| leucine | 0.1 |
| thiamine | 0.15 |
| MgSO₄ | 10 ml of 1M |
| PPG2000 | 5 ml; |
| autoclave in fermenter in 8 liters of H2O. | |

When cool, combine solutions A, B and C aseptically in the fermenter.

### PURIFICATION OF THE 20,000 DALTON PROTEIN

The frozen paste of bacterial cells (200 g) was suspended in 2 liters of 50 mM Tris (hydroxymethyl) aminomethane buffer at pH 8.0 which contained 10 mM ethylenediaminetetraacetate (EDTA) and 1 mM phenylmethylsulfonyl fluoride (PMSF). This suspension was then stirred at room temperature until uniform. The bacterial cells were subsequentially lysed by adding one gram of lysozyme to the suspension and stirring it for an additional 30 min.

When the cells were lysed, the suspension became viscous because of the released DNA. Therefore, the suspension was stirred for 30 min with deoxyribonuclease I (10 mg) in the presence of 50 mM Mg(Cl)₂, whereupon the viscosity of the suspension decreased. The suspension was clarified by centrifugation at 7,000 x g for 45 min.

The pellet resulting from the centrifugation was extracted in 8 M urea buffered with 50 mM Tris at pH 8.0. The urea was freshly deionized just prior to its use. The urea extract was diluted with an equal volume of distilled water and brought to 23% saturation with ammonium sulfate at room temperature. The precipitated protein was removed by centrifugation at 7,000 x g for 45 min.

The clarified supernatant fluid was subjected to a low pH precipitation by dialyzing it overnight at 4°C against a solution of 20 mM piperazine and 4 M urea at pH 4.0. CHAPS (3-[(cholamidopropyl)-dimethylammonio]-1-propanesulfonate) was added to the dialysate at a concentration of 5 mM. Precipitated protein was removed by centrifuging the suspension at 7,000 x g for 45 min at 4°C.

The clarified supernatant fluid was brought to 23% saturation with ammonium sulfate at 4°C. The precipitated protein was again removed by centrifugation as above. The resulting pellet was dissolved at room temperature in 100 ml of a solution consisting of 20 mM Bis-Tris Propane (1,2-bis [tris (hydroxymethyl)-methylamino]-propane) and 6.0 M urea at pH 7.0. The resulting solution was pumped through a 3 liter (9 x 47 cm) QFF sepharose column at a flow rate of 1.8 l/h to bind the recombinant protein (r-protein) to the OFF sepharose (a strong anion exchange resin). However, the recombinant protein did not bind therefore it was recovered in fluid that flowed through the column. The other bacterial proteins did bind, however, thus considerable purification was achieved.

The r-protein was concentrated in the eluent by first diluting the urea to 4 M and then adding ammonium sulfate to a concentration of 170 g/l. The precipitated r-protein was collected by centrifuging as before except at room temperature. The resulting pellet was dialyzed exhaustively against distilled water and lyophilized. The yield of r-protein was 0.74 g.

The r-protein gave one predominant band on polyacrylamide gel electrophoresis of a protein with a molecular weight of about 22,000 daltons. The lyophilized preparation of the protein was judged as greater than 95% free of contaminating proteins, by this criterion. The amino acid composition of the 22,000 dalton r-protein agreed, within experimental error, agreed with the amino acid composition calculated from the known DNA sequence. Also, the N-terminal sequence for the purified 22,000 dalton r-protein through 5 cycles is ASMTG and is in perfect agreement with the predicted sequence except for the missing N-terminal methionine.

### DEMONSTRATION OF ANISOTROPY OF THE 20,000 DALTON PROTEIN

Solutions containing 4.7% and 9.6% of 22,000 dalton r-protein in HFIP were prepared by combining polymer and solvent in heat-sealed polyethylene containers. Intermittent vigorous mixing followed by standing overnight at room temperature produced thin, clear, readily pourable solutions with slight blue or violet casts. Samples of these solutions were invisible in the dark field optical microscope. On this basis, the solutions were characterized as isotropic.

A solution containing 14.6% of 22,000 dalton r-protein in HFIP was prepared by combining 0.2582 g polymer and 1.5149 g of solvent in a heat-sealed polyethylene container. Intermittent vigorous mixing followed by standing overnight at room temperature produced a nearly clear but faintly translucent solution. The solution showed some indication of yield-stress rheology, but a sample failed to show brightness in the dark field optical microscope. On this basis, the solution was characterized as isotropic close to the critical concentration for anisotropy.

A solution containing 15.4% of 22,000 dalton r-protein in HFIP was prepared by diluting a previously-prepared 29.5% solution with additional solvent. Intermittent vigorous mixing following by standing overnight at room temperature produced a translucent, pasty solution with yield-stress rheology and a faint blue or lavender cast. A sample of the solution was examined in the dark field optical microscope and was found to be birefringent, showing zones which brightened and darkened as the sample was rotated in the plane of the microscope stage. On the basis of its rheology, overall appearance, and optical properties, the solution was characterized as anisotropic (liquid crystalline).

A solution containing 19.5% of 22,000 dalton r-protein in HFIP was prepared by combining 0.3070 g polymer and 1.2678 g solvent in a heat-sealed polyethylene container. Intermittent vigorous mixing followed by standing four days a room temperature produced a thick, pasty, translucent solution with a pronounced yield-stress (Bingham plastic) rheology. A sample of the solution was examined in the dark field optical microscope and was found to be birefringent, showing zones which brightened and darkened as the sample was rotated in the plane of the microscope stage. On the basis of its rheology, overall appearance, and optical properties, the solution was characterized as anisotropic (liquid crystalline).

### PRODUCTION OF A FIBER WITH THE 20,000 DALTON PROTEIN

A solution containing 19.5 wt% of the 22,000 dalton r-protein in HFIP was prepared by adding the solvent to the dry polymer in a heat-sealed polyethylene packet, mixing thoroughly by kneading by hand, and then mixing intermittently for a period of four days. The resulting solution showed the translucent, opalescent appearance and yield-stress (Bingham plastic) rheology often associated with anisotropic solutions. A sample of the solution was placed between crossed polarizing filters (set 90 degrees apart) in the optical train of a light microscope and was examined in the resulting dark field. The sample was birefringent, showing zones which brightened and darkened as the sample was rotated in the plane of the microscope stage. The solution was characterized as anisotropic (liquid crystalline) on the basis of its rheology, overall appearance, and optical properties.

The solution was loaded into a syringe fitted with 50, 325, 325, and 50 mesh screens in succession. The syringe was capped and centrifuged to disengage air bubbles trapped in the solution. A syringe pump was then used to force the solution through the screen pack and out of the syringe through a 0.005 inch diameter x 0.010 inch length orifice in a stainless steel fitting directly into a beaker of room temperature methanol. The syringe pump speed was set to deliver 0.0034 ml/min of solution. The white, opaque filament which formed as the solution was extruded into the methanol was allowed to fall freely and to coil on itself at the bottom of the beaker.

After at least 1 h, coagulation in the methanol, the filament was removed and allowed to dry in air at room temperature to produce an 80 denier fiber with a tenacity of 0.30 gpd, an elongation of 2%, and an initial modulus of 15.5 gpd.

Alternatively, after coagulation in methanol for 1 h, the filament was drawn up to 2.5 times its original length while immersed in methanol and was then allowed to dry in air at room temperature to produce a 45 denier fiber with a tenacity of 0.40 gpd, an elongation of 2.5%, and an initial modulus of 20 gpd.

### CROSS-BETA SHEET MICROFIBRILLAR STRUCTURE OF THE 20,000 DALTON PROTEIN

Protein of the sample was dissolved in water and cast to make a film. A sample from the cast film was subjected to an x-ray powder diffraction analysis. The results showed a diffraction pattern that corresponds to the structure that a polypeptide is anticipated to form. Radial averaging corresponded to a what would be expected if cross-beta sheets or microfibrils were lying unoriented on a surface.

As many differing embodiments of this invention may be made without departing from the spirit and scope thereof, it is to be understood that this invention is not limited to the specific embodiments exemplified except as defined by the appended claims.

## Claims

1. A synthetic DNA sequence encoding a protein, said protein formed from 14 to 52 multiples of

SGLDFDNNALRIKLG (SEQ ID NO:3).

2. The DNA sequence of Claim 1 operably linked in a vector and expressed as a polypeptide expression product of a transformed bacteria.

3. The sequence of Claim 2 wherein the vector is pRHF-1 or pRHf-4 as defined in claim 16.

4. The sequence of Claim 2 wherein the vector includes:
(a) a strong conditional promoter upstream of the synthetic gene;
(b) a sequence downstream of the promoter that would contain the signals necessary for the translation start of the synthetic gene; and
(c) a restriction site downstream of the translational start that would allow the insertion of the concatemeric oligonucleotide in the correct orientation and proper reading frame such that translation will result in protein with the desired amino acid sequence.

5. The polypeptide expression product(s) of Claim 2.

6. The polypeptide expression product of Claim 5 in the form of a regular, chain-folded cross-beta sheet.

7. The polypeptide expression product of Claim 5 in the form of an aggregate of multiple aligned copies of cross-beta sheets.

8. A solution of the product(s) of Claim 5.

9. The solution of Claim 8, in a solvent selected from the group consisting of hexafluoroisopropanol, hexafluoroisopropanol/urea, dichloroacetic acid, formic acid, formic acid/lithium chloride and trifluoroacetic acid.

10. The solution of Claim 9, where the solvent is selected from hexafluoroisopropanol and hexafluoroisopropanol/urea.

11. The product of Claim 8 used as a film.

12. The product of Claim 5 used in a non-linear optical apparatus.

13. The product of Claim 5 used as a fiber.

14. A process for producing a desired protein comprising:
(a) transforming a host bacterium with a vector comprising a DNA sequence of Claim 1;
(b) culturing the transformed host bacteria;
and (c) recovering the protein.

15. The process of Claim 14, comprising adding an inducer to the cultured bacteria after step (b).

16. The process of Claim 14 wherein the vector is pRHF-1 or pRHF-4 containing
(a) a strong conditional promoter upstream of the synthetic gene;
(b) a sequence downstream of the promoter containing means for signaling the translation start of the synthetic gene; and
(c) a restriction site downstream of the translational start that would allow the insertion of a concatemeric oligonucleotide in the correct orientation and reading frame such that the translation will result in a protein with a desired amino acid sequence.

17. The process of Claim 16 wherein the conditional promoter is selected from a bacteriophage lambda P_{L} promoter and a bacteriophage T7 gene 10 promoter.

18. The process of Claim 16 wherein the host bacterium is an *E. coli* strain.

19. The process of Claim 18 wherein the host is an *E. coli* strain that is defective for *rec A* gene.

20. The process of Claim 19 wherein the host is selected from DH5Alac and JM109.

21. The process of Claim 16 wherein the host is an *E. coli* strain BL21 or its derivatives.

22. The process of Claim 17 wherein the promoter is a bacteriophage lambda P_{L} promoter.

23. The process of Claim 22 wherein the host contains a lambda cI repressor gene.

24. The process of Claim 23 wherein the host is *E. coli* strain OR1265.

25. A solution of the product of Claim 6.

26. A solution of the product of Claim 7.

27. The solution of Claim 26 used as a liquid crystal.

## Patentansprüche

1. Eine synthetische DNA Sequenz, die ein Protein codiert, wobei das Protein gebildet ist aus 14 bis 52 Mehrfachen von SGLDFDNNALRIKLG (SEQ ID NR:3).

2. DNA Sequenz nach Anspruch 1, operabel in einen Vektor gekoppelt und als ein Polypeptidexpressionsprodukt einer transformierten Bakterie exprimiert.

3. Sequenz nach Anspruch 2, wobei der Vektor pRHF-1 oder pRHF-4, wie in Anspruch 16 definiert, ist.

4. Sequenz nach Anspruch 2, wobei der Vektor umfaßt:
(a) einen starken konditionalen Promotor stromaufwärts von dem synthetischen Gen;
(b) eine Sequenz stromabwärts des Promotors, die die für den Translationsstart des synthetischen Gens notwendigen Signale enthalten würde; und
(c) eine Restriktionsstelle stromabwärts des Translationsstarts, die die Insertion des konkatemeren Oligonucleotids in das korrekte Orientierungs- und richtige Leseraster erlauben würde, so daß Translation zu Protein mit der gewünschten Aminosäurensequenz führt.

5. Polypeptidexpressionsprodukt(e) nach Anspruch 2.

6. Polypeptidexpressionsprodukt nach Anspruch 5 in der Form einer regulären, kettengefalteten Kreuz-beta-Lage.

7. Polypeptidexpressionsprodukt nach Anspruch 5 in der Form eines Aggregats mehrfach ausgerichteter Kopien von Kreuz-beta-Lagen.

8. Lösung des (der) Produkts (Produkte) nach Anspruch 5.

9. Lösung nach Anspruch 8 in einem Lösungsmittel, ausgewählt aus der Gruppe bestehend aus Hexafluorisopropanol, Hexafluorisopropanol/Harnstoff, Dichloressigsäure. Ameisensäure, Ameisensäure/Lithiumchlorid und Trifluoressigsäure.

10. Lösung nach Anspruch 9, wobei das Lösungsmittel ausgewählt ist aus Hexafluorisopropanol und Hexafluorisopropanol/Harnstoff.

11. Produkt nach Anspruch 8, verwendet als ein Film.

12. Produkt nach Anspruch 5, verwendet in einer nicht linearen optischen Vorrichtung.

13. Produkt nach Anspruch 5, verwendet als eine Faser.

14. Vorrichtung zum Herstellen eines gewünschten Proteins, umfassend:
(a) Transformieren eines Wirtsbakteriums mit einem Vektor, enthaltend eine DNA Sequenz nach Anspruch 1;
(b) Kultivieren der transformierten Wirtsbakterien: und
(c) Gewinnen des Proteins.

15. Verfahren nach Anspruch 14, umfassend Hinzufügen eines Induktors zu den kultivierten Bakterien nach Stufe (b).

16. Verfahren nach Anspruch 14, wobei der Vektor pRHF-1 oder pRHF-4 ist, enthaltend
(a) einen starken konditionalen Promotor stromaufwärts des synthetischen Gens;
(b) eine Sequenz stromabwärts des Promotors, enthaltend Mittel zum Signalisieren des Translationsstarts des synthetischen Gens; und
(c) eine Restriktionsstelle stromabwärts des Translationsstarts, die die Insertion eines konkatemeren Oligonukleotids in das korrekte Orientierungs- und Leseraster erlauben würde, so daß die Translation zu einem Protein mit einer gewünschten Aminosäurensequenz führt.

17. Verfahren nach Anspruch 16, wobei der konditionale Promotor ausgewählt ist aus einem Bakteriophagen lambda P_{L} Promotor und einem Bakteriophagen T7 Gen 10 Promotor.

18. Verfahren nach Anspruch 16. wobei das Wirtsbakterium ein E. coli Stamm ist.

19. Verfahren nach Anspruch 18, wobei der Wirt ein E. coli Stamm ist. der defekt in Bezug auf rec A Gen ist.

20. Verfahren nach Anspruch 19, wobei der Wirt ausgewählt ist aus DH5Δlac und JM109.

21. Verfahren nach Anspruch 16, wobei der Wirt ein E. coli Stamm BL21 oder dessen Derivate ist.

22. Verfahren nach Anspruch 17, wobei der Promotor ein Bakteriophagen lambda P_{L} Promotor ist.

23. Verfahren nach Anspruch 22, wobei der Wirt ein lambda cI Repressor Gen enthält.

24. Verfahren nach Anspruch 23, wobei der Wirt E. coli Stamm OR1265 ist.

25. Lösung des Produkts nach Anspruch 6.

26. Lösung des Produkts nach Anspruch 7.

27. Lösung nach Anspruch 26, verwendet als ein Flüssigkristall.

## Revendications

1. Séquence d'ADN synthétique codant pour une protéine, ladite protéine étant formée de 14 à 52 multiples de SGLDFDNNALRIKLG (SEQ ID NO:3).

2. Séquence d'ADN selon la revendication 1 liée de façon opérationnelle dans un vecteur et exprimée sous forme de produit d'expression polypeptidique d'une bactérie transformée.

3. Séquence d'ADN selon la revendication 2 dans laquelle le vecteur est pRHF-1 ou pRHF-4 selon la revendication 16.

4. Séquence d'ADN selon la revendication 2 dans laquelle le vecteur inclut:
(a) un promoteur conditionnel puissant en amont du gène synthétique:
(b) une séquence en aval du promoteur contenant les signaux nécessaires pour le début de la traduction du gène synthétique; et
(c) un site de restriction en aval du début de la traduction permettant l'insertion de l'oligonucléotide concatémérique dans l'orientation correcte et le cadre de lecture approprié, de sorte que la traduction va avoir comme résultat une protéine avec la séquence voulue en acides aminés.

5. Produit(s) d'expression polypeptidique(s) selon la revendication 2.

6. Produit d'expression polypeptidique selon la revendication 5 sous la forme de feuillet régulier béta-croisé replié en chaîne.

7. Produit d'expression polypeptidique selon la revendication 5 sous la forme d'un agrégat de copies multiples alignées de feuillets béta-croisé.

8. Solution du (des) produits selon la revendication 5.

9. Solution selon la revendication 8, dans un solvant choisi parmi le groupe constitué par l'alcool hexafluoroisopropylique, l'alcool hexafluoroisopropylique/urée, l'acide dichloracétique, l'acide formique, l'acide formique/chlorure de lithium et l'acide trifluoroacétique.

10. Solution selon la revendication 9, dans laquelle le solvant est choisi parmi l'alcool hexafluoroisopropylique et l'alcool hexafluoroisopropylique/urée.

11. Produit selon la revendication 8 utilisé en tant que film.

12. Produit selon la revendication 5 utilisé dans un appareil optique non-linéaire.

13. Produit selon la revendication 5 utilisé en tant que fibre.

14. Processus pour la production d'une protéine voulue comprenant:
(a) transformation d'une bactérie hôte avec un vecteur comprenant une séquence d'ADN selon la revendication 1;
(b) cultivation de la bactérie hôte transformée: et
(c) récupération de la protéine.

15. Processus selon la revendication 14, comprenant l'addition d'un inducteur aux bactéries cultivées après l'étape (b).

16. Processus selon la revendication 14 dans lequel le vecteur est pRHF-1 où pRHF-4 contenant
(a) un promoteur conditionnel puissant en amont du gène synthétique;
(b) une séquence en aval du promoteur contenant un moyen pour signaler le début de la traduction du gène synthétique; et
(c) un site de restriction en aval du début de la traduction permettant l'insertion de l'oligonucléotide concatémérique dans l'orientation correcte et le cadre de lecture approprié de sorte que la traduction va avoir comme résultat une protéine avec la séquence voulue en acides aminés.

17. Processus selon la revendication 16 dans lequel le promoteur conditionnel est choisi parmi un promoteur de bactériophage lambda P_{L} et un promoteur de bactériophage T7 gène 10.

18. Processus selon la revendication 16 dans lequel la bactérie hôte est une souche d'E. coli.

19. Processus selon la revendication 18 dans lequel l'hôte est une souche d'E. coli avec un gène rec A défectueux.

20. Processus selon la revendication 19 dans lequel l'hôte est choisi parmi DH5Δlac et JM109.

21. Processus selon la revendication 16 dans lequel l'hôte est une souche d'E. coli BL21 ou ses dérivés.

22. Processus selon la revendication 17 dans lequel le promoteur est un promoteur de bactériophage lambda P_{L}.

23. Processus selon la revendication 22 dans lequel l'hôte contient un gène de répresseur lamba cI.

24. Processus selon la revendication 23 dans lequel l'hôte est une souche d'E. coli OR1265.

25. Solution du produit de la revendication 6.

26. Solution du produit de la revendication 7.

27. Solution de la revendication 26, utilisée comme un cristal liquide.
